# EUROPEAN PATENT APPLICATION

(11) **EP 0 917 855 A1**
(43) Date of publication of application: **26.05.1999**
(21) Application number: 98121579.1
(22) Date of filing: 19.11.1998
(51) Int. Cl.: A61B 6/00, A61B 6/03

(54) **X-Ray photographing apparatus and method capable of performing computerized tomography using C-Arm**

(30) Priority: 24.11.1997 KR 9762386
(71) Applicant: MEDISON CO., LTD., Kangwon-do 250-870 (KR)
(72) Inventor: Lee, Min Hwa, Kangnam-gu, Seoul (KR); Kim, Yeong Mo, Seocho-gu, Seoul (KR)
(74) Representative: Reinhard - Skuhra - Weise & Partner

(57) **Abstract**

In an X-ray photographing apparatus including a C-arm, an X-ray photographing apparatus and method capable of performing a computerized tomography (CT) using the C-arm is provided to obtain a plurality of X-ray planar images by moving the C-arm and form a tomogram and a three-dimensional image using the plurality of the planar images. The apparatus of the present invention obtains the plurality of X-ray planar images photographed by adjusting angles of the C-arm in turn, and forms the plurality of X-ray planar images into a tomogram and a three-dimensional image using a predetermined signal processing algorithm, thereby to obtain an effect of performing a CT function with an inexpensive C-arm X-ray photographing apparatus without using an expensive CT apparatus.

## Description

### BACKGROUND OF THE INVENTION

### 1. Field of the Invention

The present invention relates to a C-arm X-ray photographing apparatus and method capable of performing a computerized tomography (CT) using a C-arm, and more particularly, to a C-arm X-ray photographing apparatus and method capable of performing a computerized tomography (CT), in which the C-arm is adjusted in order to obtain a plurality of X-ray planar images photographed at different angles from one another in an X-ray photographing apparatus, and a tomogram and a three-dimensional image can be formed of the plurality of the X-ray planar images.

### 2. Description of the Related Art

Generally, the X-ray photographing apparatus produces an X-ray image with respect to a difference of absorptive rates between various organs in the human body, using the X-ray having different absorptive properties according to substances. The X-ray image is a silhouette picture of various structures in the human body which are projected on a film. The X-ray photographing apparatus is used for non-destructive testing in order to disclose defects of the internal structure together with diagnosis of the large intestine, the stomach and phthisis.

A CT apparatus which is a further developed X-ray photographing apparatus irradiates a certain amount of X-rays on a portion to be photographed in the human body, measures the amount of the X-rays transmitting through the photographed portion with an X-ray detector. records the result in a memory, obtains an absorptive rate of the X-ray in each portion to be photographed in the human body using a computer, and reconstructs an image according to the obtained absorptive rate. An image obtained by the CT apparatus is not a general X-ray picture which is represented by accumulating three-dimensional information of the inside of an object on a two-dimensional film. The image obtained by the CT apparatus shows all information with respect to an arbitrary selected cross-section quantitatively, to thereby contribute to allow users to accurately diagnose various facts which are difficult to be found by the general X-ray picture. However, there is a drawback that the CT apparatus is more expensive than the X-ray photographing apparatus.

### SUMMARY OF THE INVENTION

To solve the above problems, it is an object of the present invention to provide a C-arm X-ray photographing apparatus and method capable of performing a computerized tomography (CT) using a C-arm in which a tomogram and a three-dimensional image can be formed using X-ray planar images photographed in an X-ray photographing apparatus including a C-arm.

To accomplish the above object of the present invention, there is provided a C-arm X-ray photographing apparatus for photographing an object using an X-ray generator and an X-ray detector connected with a C-arm, comprising:
memory means for storing planar image data of the object photographed at various angles; and image forming means for forming the planar image data to a tomogram or a three-dimensional image using a predetermined image construction algorithm.

To achieve the object of the present invention, there is provided a method for obtaining an image in a C-arm X-ray photographing apparatus which photograshes an object using an X-ray generator and an X-ray detector connected with the C-arm, comprising the steps of:
(a) photographing the object at various angles using an X-ray photographing apparatus including a C-arm;
(b) acquiring a plurality of X-ray planar images photographed at the various angles; and
(c) forming a tomogram using the plurality of X-ray planar images.

To achieve the object of the present invention, the method for obtaining a tomogram in a C-arm X-ray photographing apparatus further comprises the step of forming a three-dimensional image using the tomogram formed in step (c).

### BRIEF DESCRIPTION OF THE DRAWINGS

The preferred embodiment is described with reference to the drawings wherein:
Fig. 1 is a perspective view showing a C-arm X-ray photographing apparatus capable of performing a computerized tomography according to a preferred embodiment of the present invention;
Figs. 2A and 2B show states where a C-arm of Fig. 1 is moved; and
Fig. 3 is a block diagram shoving a structure of the apparatus shown in Fig. 1.

### DETAILED DESCRIPTION OF THE PREFERRED EMBODIMENT

A preferred embodiment of the present invention will be described with reference to the accompanying drawings.

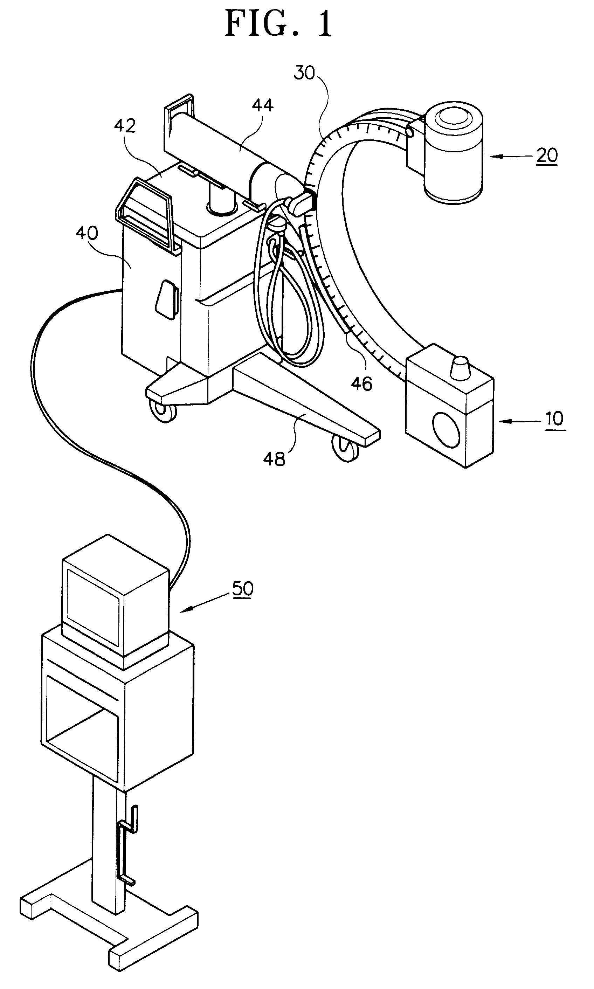

Fig. 1 shows a C-arm X-ray photographing apparatus including a C-arm according to a preferred embodiment of the present invention. The X-ray photographing apparatus includes an X-ray generator 10 for generating an X-ray and an X-ray detector 20 for detecting the generated X-ray. The X-ray generator 10 and the X-ray detector 20 are connected to a C-arm 30 having a form of alphabet C. The X-ray photographing apparatus also includes a main body 40 having a control circuit for controlling movement of the C-arm 30 and a circuit for signal-processing the photographed X-ray image. The main body 40 includes a complex movement shaft 44. The C-arm 30 can move up and down, left and right and rotate centering on the complex movement shaft 44. That is, the C -arm 30 can move in all directions of angles. A movement support plate 46 which is engaged with the C-arm 30 is located at the complex movement shaft 44.

The main body 44 also includes a control panel 42 on its upper end, and a movement wheel unit 48 for enabling the X-ray photographing apparatus to move up and down, left and right and rotate on its lower end. The main body 40 and a TV monitor 50 in the X-ray photographing apparatus are connected with each other by cable. The TV monitor 50 displays images which have been signal-processed by the control circuit incorporated in the main body 40.

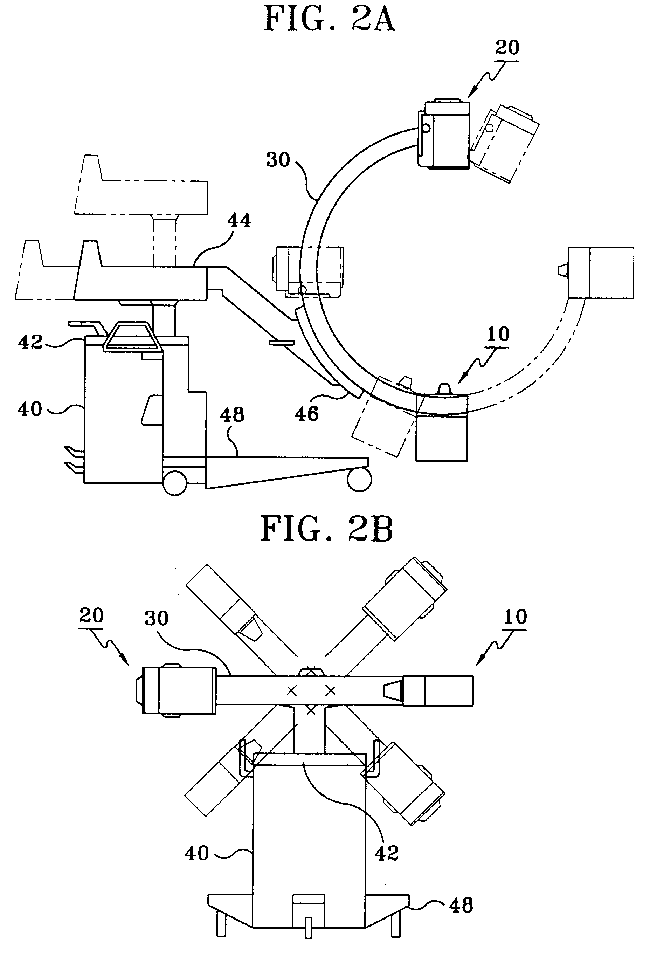

Figs. 2A and 2B show the states where the C-arm 30 of Fig. 1 moves up and down, and left and right and rotates, in which Fig. 2A is a side view of the apparatus shown in Fig. 1 and Fig. 2B is a rear view thereof. As shown in Figs. 2A and 2B, since the C-arm 30 can rotate at all angles, an X-ray photographing is possible at all angles.

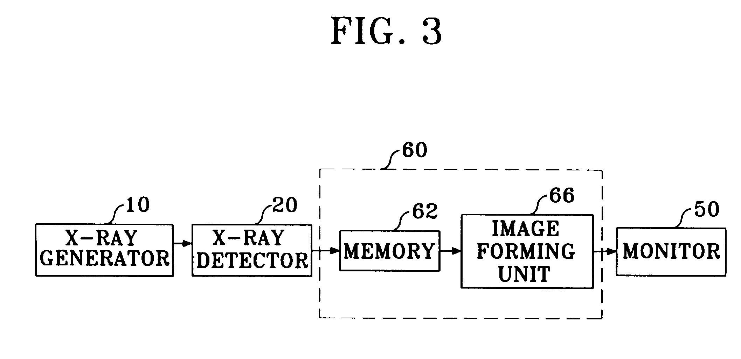

Fig. 3 is a block diagram showing the structure of the Fig. 1 apparatus. The X-ray generator 10 includes a high-voltage generator (not shown) and generates a stable X-ray through an ordinary inverting method. The X-ray detector 20 further includes an image intensifier (II) which can obtain a more clear image with a small amount of X-rays or a digital radiography (DR) or a digital fluorocopy (DF) for digital-processing a Roentgen ray image. Here, since respective structures of the X-ray generator 10 and the X-ray detector 20 are known in the art, the detailed description thereof will be omitted. The planar image data detected in the X-ray detector 20 is applied to a controller 60 incorporated in the main body 40. The controller 60 includes a memory 62 for storing X-ray planar image data therein, and an image forming unit 66 for forming a tomogram and a three-dimensional image using the X-ray planar image data stored in the memory 62. The TV monitor 50 displays the tomogram and the three-dimensional image formed in the image forming unit 66 thereon.

In Fig. 3, an X-ray photographer such as a doctor and a medical engineer manipulates the control panel 42 attached to the main body 40 in order to obtain a plurality of X-ray planar images, and rotates the C-arm 30 from a predetermined reference angle by a certain angle as shown in Figs. 2A and 2B. The positions of the X-ray generator 10 and the X-ray detector 20 are changed by the angle-adjusted C-arm. Thus, X-ray planar images photographed at various angles can be obtained. The memory 62 stores a plurality of the X-ray planar images photographed at various angles. The image forming unit 66 forms the tomogram using the plurality of the X-ray planar images stored in the memory 62, and also forms the three-dimensional image using the formed tomogram. Here, the tomogram and three-dimensional image forming is performed by using a well-known CT or MRI or an ultrasonic three-dimensional image forming algorithm. The TV monitor 50 displays the formed tomogram and three-dimensional image thereon.

As described above, the C-arm X-ray photographing apparatus according to the present invention forms a tomogram and a three-dimensional image using a plurality of X-ray planar images photographed at various angles by adjusting the C-arm, and provides an effect of performing a CT with an inexpensive X-ray photographing apparatus, without using an expensive CT photographing apparatus.

While only a certain embodiment of the invention has been specifically described herein, it will be apparent that numerous modifications may be made thereto without departing from the spirit and scope of the invention.

## Claims

1. A C-arm X-ray photographing apparatus for photographing an object using an X-ray generator and an X-ray detector connected with a C-arm, comprising:
memory means for storing planar image data of the object photographed at various angles; and
image forming means for forming the planar image data to a tomogram or a three-dimensional image using a predetermined image construction algorithm.

2. A method for obtaining an image in a C-arm X-ray photographing apparatus which photograshes an object using an X-ray generator and an X-ray detector connected with the C-arm, comprising the steps of:
(a) photographing the object at various angles using an X-ray photographing apparatus including a C-arm;
(b) acquiring a plurality of X-ray planar images photographed at the various angles; and
(c) forming a tomogram using the plurality of X-ray planar images.

3. The method for obtaining a tomogram in a C-arm X-ray photographing apparatus according to claim 2, further comprising the step of forming a three-dimensional image using the tomogram formed in step (c).
